# EUROPÄISCHE PATENTANMELDUNG

(11) **EP 1 312 386 A2**
(43) Veröffentlichungstag der Anmeldung: **21.05.2003**
(21) Anmeldenummer: 02024181.6
(22) Anmeldetag: 30.10.2002
(51) Int. Cl.: A61M 1/16

(54) **Behältnis mit Bestandteilen eines Konzentrats, insbesondere mit Bestandteilen eines sauren Konzentrats für eine Dialysierflüssigkeit sowie Verfahren und Vorrichtung zum Herstellen eines Konzentrats**

(30) Priorität: 30.10.2001 DE 10153063
(71) Anmelder: GML Aktiengesellschaft für Medizintechnik und Logistikmanagement, 63263 Neu-Isenburg (DE)
(72) Erfinder: Kaupa, Paul, 64331 Weiterstadt (DE)
(74) Vertreter: Stoffregen, Hans-Herbert, Dr. Dipl.-Phys. Patentanwalt

(57) **Zusammenfassung**

Die Erfindung bezieht sich auf ein Behältnis mit Bestandteilen eines Konzentrats, insbesondere eines sauren Konzentrats für eine Dialysierflüssigkeit sowie Verfahren und Vorrichtung zum Aufbereiten eines Konzentrats, insbesondere einer Dialysierflüssigkeit. Das Behältnis (10) wiest eine erste und eine zweite Einlass-/Auslassöffnung (16, 18) auf. Um eine Volumen- und Gewichtsreduktion zu erreichen ist vorgesehen, dass das Behältnis (10) selbst als Mischbehältnis mit einem Volumen derart ausgebildet ist, dass eine definierte Flüssigkeitsmenge zur Lösung der Bestandteile (12) aufnehmbar ist.

## Beschreibung

Die Erfindung bezieht sich auf ein Behältnis mit Bestandteilen eines Konzentrats, insbesondere mit Bestandteilen eines sauren Konzentrats für eine Dialysierflüssigkeit, wobei das Behältnis eine erste und eine zweite Einlass-/Auslassöffnung aufweist. Ferner bezieht sich die Erfindung auf ein Verfahren und eine Vorrichtung zum Herstellen eines Konzentrats, insbesondere eines sauren Konzentrats für eine Dialysierflüssigkeit, wobei in einem Behältnis transportierbare Bestandteile mit einer Flüssigkeit wie Wasser vermischt werden.

Ein Behältnis sowie ein Verfahren und eine Vorrichtung der zuvor genannten Art sind in der WO 01/02036 A1 beschrieben. Dabei ist vorgesehen, dass die zur Herstellung einer Dialysierflüssigkeit für eine Dialysebehandlung wesentlichen Bestandteile des sauren Konzentrats in dem Behältnis in so hoher Konzentration vorliegen, dass sie nur partiell in Wasser gelöst sind, während erhebliche Anteile der obigen Bestandteile ungelöst bleiben und mit der Lösung eine Aufschlemmung bilden. Dabei ist vorgesehen, dass das Behältnis als Kartusche ausgebildet ist.

Eine Vorrichtung zum Aufbereiten des sauren Konzentrats zeichnet sich dadurch aus, dass eine lösbar eingesetzte Kartusche mit den als Aufschlemmung vorliegenden Bestandteilen eines sauren Dialysekonzentrats, ein Batch-Gefäß, verbindende Leitungen, einen Wasserzulauf und einen -ablauf sowie eine Pumpe zur Zirkulation des zugeführten Wassers bzw. des sich bildenden Konzentrats vorgesehen sind, um die festen Bestandteile vollständig aufzulösen und homogen im Zirkulationssystem zu verteilen. Dabei ist vorgesehen, dass eine Flüssigkeit wie Wasser vorzugsweise von unten in die mit den sauren Bestandteilen gefüllte Kartusche eintritt. Dabei sind die Anschlüsse des Behältnisses an gegenüberliegenden Seiten angebracht. Beim Zu- und Ablauf der Kartusche sind jeweils Filter vorgesehen, die das Austreten ungelöster Bestandteile aus der Kartusche verhindern. Nach Einströmen von Wasser in die Kartusche fließt die entstehende Lösung in ein Batch-Gefäß. Ein Füllstandssensor beendet den Wasserzulauf. Ein Füllstandssensor ermöglicht die Festlegung unterschiedlicher, genau definierter Verdünnungsverhältnisse, wobei das gesamte Batch-Volumen der Summe der Volumina von Batch-Gefäß und Kartusche und dem zugehörigen Schlauchsystem entspricht.

Mit anderen Worten ist zwingend ein separates Batch-Gefäß vorgesehen, wodurch der Apparateaufwand der Vorrichtung aufwendig wird.

Davon ausgehend liegt der vorliegenden Erfindung das Problem zu Grunde, ein Behältnis sowie ein Verfahren und eine Vorrichtung der zuvor genannten Art dahingehend weiterzubilden, dass neben Einsparungseffekten bei Transport und Lagerung des Konzentrats die Handhabung des Behältnisses sowie der Vorrichtung weiter vereinfacht wird.

Das Problem wird durch ein Behältnis dadurch gelöst, das als Mischbehältnis ausgebildet ist und ein Volumen derart aufweist, dass eine definierte Flüssigkeitsmenge zur Lösung der Bestandteile aufnehmbar ist.

Das erfindungsgemäße Behältnis zeichnet sich gegenüber dem Stand der Technik dadurch aus, dass dieses gleichzeitig als Mischbehältnis bzw. Batch-Gefäß ausgebildet ist, so dass auf ein separates Batch-Gefäß verzichtet werden kann. Dabei ist das Behältnis in seinem Volumen derart ausgebildet, dass die gesamte für die Lösung des Konzentrats notwendige Flüssigkeitsmenge von dem Behältnis aufgenommen werden kann.

Da sämtliche Bestandteile des Konzentrats in dem Behältnis in trockener Form enthalten sind, wird zudem das Volumen und das Gewicht der zu transportierenden, zu lagernden und zu handhabenden Bestandteile des Konzentrats reduziert.

In bevorzugter Ausführungsform umfasst das Behältnis einen im Wesentlichen trichterförmigen Bereich zur Aufnahme der Bestandteile des Konzentrats, an dessen spitz zulaufender Grundseite zumindest die erste Einlass-/Auslassöffnung für die Flüssigkeit und/oder die Konzentratlösung angeordnet ist.

Eine weitere Volumenreduzierung wird dadurch erreicht, dass das Behältnis als Kunststoffbeutel ausgebildet ist, vorzugsweise mit einem Fassungsvermögen von 3 bis 8 1, vorzugsweise 5 1,-wobei das Fassungsvermögen in Abhängigkeit der Behandlungszeit angepasst werden kann.

Zur Vereinfachung der Handhabung ist vorgesehen, dass das Behältnis als Einwegbeutel ausgebildet ist, d. h. dass dieser nach Befüllen mit Flüssigkeit und Mischen des Konzentrats sowie nach Ausgabe der Konzentratlösung auf einfachste Weise entsorgt werden kann.

Um eine Vermischung der Bestandteile mit der Flüssigkeit zu gewährleisten, ist die zweite Einlass-/Auslassöffnung bezogen auf die Grundseite der trichterförmigen Aufnahme in einer Höhe angeordnet, die unterhalb eines Flüssigkeitspegels des mit Flüssigkeit gefüllten Behältnisses angeordnet ist. Dabei wird die Flüssigkeit beispielsweise durch die erste Einlassöffhung in das Behältnis eingeleitet und durch die zweite Öffnung abgeleitet, sobald der Flüssigkeitsspiegel die Höhe der oberen Öffnung erreicht hat. Sodann kann ein Zirkulationskreislauf zur Auflösung der Bestandteile in der Flüssigkeit realisiert werden.

In bevorzugter Ausrührungsform ist vorgesehen, dass das Behältnis ein Beutel ist, der in einer Vorrichtung zur Aufbereitung des Konzentrats einsetzbar ist.

Weiterhin bezieht sich die Erfindung auf ein Verfahren zum Herstellen eines Konzentrats, insbesondere eines sauren Konzentrats für eine Dialysierflüssigkeit, durch Vermischen von in einem Behältnis transportierbaren Bestandteile des Konzentrats mit einer Flüssigkeit wie Wasser. Das erfindungsgemäße Verfahren sieht vor, dass als Behältnis ein solches verwendet wird, in dem die Bestandteile des Konzentrats der Flüssigkeit aufgelöst werden und in dem das fertige Konzentrat bereitgestellt wird.

Gemäß einer bevorzugten Verfahrensweise wird eine definierte Flüssigkeitsmenge in das Behältnis eingeleitet, wobei die sich in dem Behältnis bildende Lösung sodann beispielsweise mittels einer Pumpe zirkuliert wird. Dadurch wird eine vollständige Auflösung der vorzugsweise pulverförmigen Bestandteile erreicht.

Eine besonderes wirksame Durchmischung der Lösung wird erreicht, wenn bei einer Temperatur von 20 °C bis 39 °C, vorzugsweise 37 °C aufgemischt wird. Die Menge der in das Behältnis einzuleitenden Flüssigkeit kann vorteilhaft über die Hubzahl einer Kolbentaumelpumpe oder ein Durchflussmessgerät bestimmt werden.

Schließlich bezieht sich die Erfindung auf eine Vorrichtung zum Herstellen eines Konzentrats, insbesondere eines sauren Konzentrats einer Dialysierflüssigkeit. Die Vorrichtung umfasst ein in einem Kreislauf angeordnetes Behältnis, in dem die Bestandteile des Konzentrats in einer Flüssigkeit lösbar sind sowie eine in dem Kreislauf angeordnete Pumpe. Erfindungsgemäß ist vorgesehen, dass das Behältnis als Transportbehältnis für die Bestandteile ausgebildet ist und ein Volumen derart aufweist, dass eine definierte Flüssigkeitsmenge zur Lösung der Bestandteile aufnehmbar ist.

In bevorzugter Ausführungsform ist vorgesehen, dass ein Ausgang der Pumpe mit der ersten Einlass-/Auslassöffnung des Behältnisses verbunden ist und dass die zweite Einlass-/Auslassöffnung des Behältnisses vorzugsweise über ein Ventil mit einem Eingang der Pumpe verbunden ist und dass des Weiteren der Eingang der Pumpe vorzugsweise über ein Ventil mit einer Flüssigkeitsquelle und die erste Einlass-/Auslassöffnung des Behältnisses, vorzugsweise über eine weitere Pumpe mit einem Dialysegerät verbunden ist.

Die erfindungsgemäße Vorrichtung zeichnet sich durch einen besonders einfachen Aufbau aus, wobei das als Einwegartikel ausgebildete Behältnis ein Volumen derart aufweist, dass dieses sowohl als Aufbewahrungsbehältnis für die trockenen Bestandteile des Konzentrats als auch als Mischbehältnis verwendbar ist. Auf ein zusätzliches Batch-Gefäße gemäß dem Stand der Technik kann somit verzichtet werden, wodurch der Aufbau insgesamt vereinfacht wird. Besonders vorteilhaft ist zu erwähnen, dass die Vorrichtung in ein Dialysegerät integriert sein kann. Selbstverständlich kann die Vorrichtung auch als Zusatzgerät mit einem Dialysegerät verbunden sein.

Weitere Einzelheiten, Vorteile und Merkmale der Erfindung ergeben sich nicht nur aus den Ansprüchen, den diesen zu entnehmenden Merkmalen - für sich und/oder in Kombination -, sondern auch aus der nachfolgenden Beschreibung eines den Zeichnungen zu entnehmenden bevorzugten Ausführungsbeispieles.

Es zeigen:
- Fig. 1: eine schematische Darstellung eines Behältnisses mit Bestandteilen eines Konzentrats, insbesondere mit Bestandteilen des sauren Konzentrats für eine Dialysierflüssigkeit und
- Fig. 2: eine schematische Darstellung einer Vorrichtung zur Aufbereitung eines Konzentrats, insbesondere des sauren Konzentrats einer Dialysierflüssigkeit.

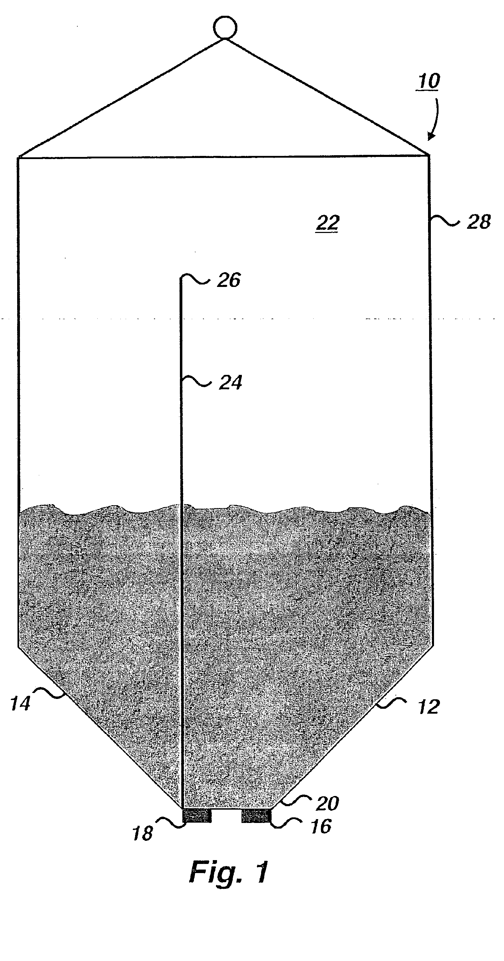

Fig. 1 zeigt in rein schematischer Darstellung ein Behältnis 10 mit trockenen, vorzugsweise pulverförmigen Bestandteilen 12 eines Konzentrats. Bei den Bestandteilen 12 handelt es sich im Folgenden um Bestandteile eines sauren Konzentrats für eine Dialysierflüssigkeit. In dem Behältnis 10 ist die für eine Dialysebehandlung an einem Patienten erforderliche Quantität der Inhaltsstoffe des sauren Konzentrats aufgenommen. Das als flexibler Kunststoffbeutel ausgebildete Behältnis umfasst einen im Wesentlichen trichterförmigen Bereich 14, in dem die Bestanteile 12 aufgenommen sind. Des Weiteren umfasst das Behältnis 10 ein Volumen, in dem einerseits die Bestandteile 12 mit einem Gewichtsanteil von etwa 1200 g bis 1400 g vorliegen, und andererseits Flüssigkeit in einer Menge von ca. 3 1 bis 8 1, vorzugsweise 5 1 aufgenommen wird. Ferner umfasst das Behältnis 10 eine erste Einlass-/Auslassöffnung 16 sowie eine zweite Einlass-/Auslassöffnung 18, die an einer spitz zulaufenden Grundseite 20 des trichterförmigen Bereichs 14 des Behältnisses angeordnet sind. Dabei ist vorgesehen, dass die zweite Einlass-/Auslassöffnung 18 im Inneren 22 des Behältnisses 10 eine Leitung 24 mit einer Öffnung 26 aufweist, die mit Bezug zu der Grundseite 20 des trichterförmigen Bereichs 14 in einem oberen Abschnitt 28 des Behältnisses angeordnet ist. Dabei kann die Leitung 24 beispielsweise mit einer Seitenwandung des als Folienbeutel ausgebildeten Behältnisses 10 verbunden wie verschweißt sein.

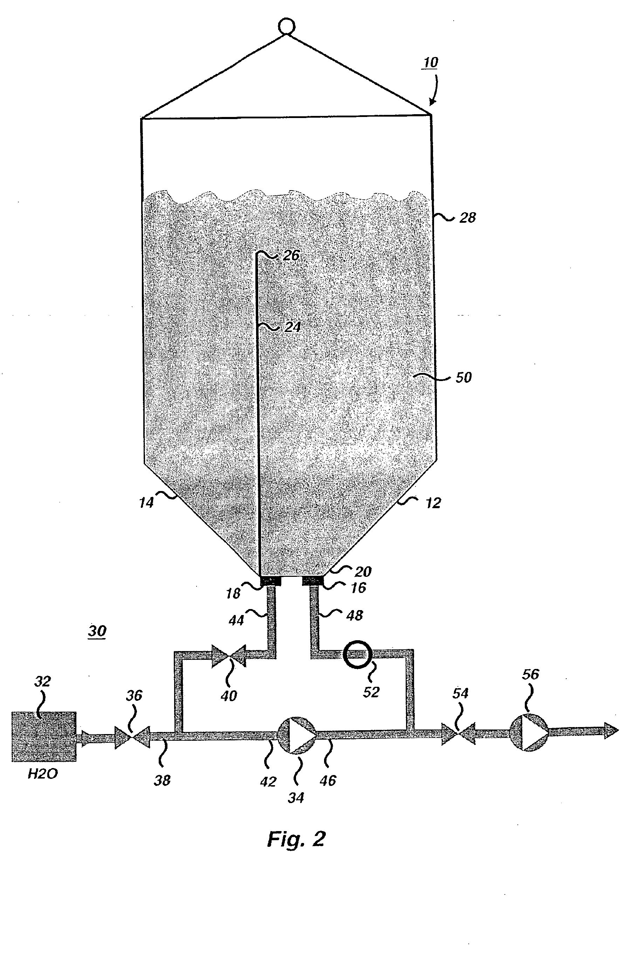

In dem in Fig. 1 dargestellten Zustand wird das Behältnis 10 angeliefert und kann sodann in eine in Fig. 2 rein schematisch dargestellte Vorrichtung 30 zur Aufbereitung des sauren Konzentrats 12 für eine Dialysierflüssigkeit eingesetzt werden. Die Vorrichtung 30 kann als Bestandteil einer Dialysevorrichtung (nicht dargestellt) ausgebildet sein. Aus einem Flüssigkeitsreservoir 32 wird eine Flüssigkeit wie Wasser geeigneter Qualität über eine Pumpe 34 und die erste Öffnung 16 von unten in das Behältnis 10 eingeleitet, wodurch eine Vermischung der trockenen Bestandteile 12 mit der Flüssigkeit stattfindet. Die Flüssigkeit wird in einer vorbestimmten Menge in das Behältnis 10 eingeleitet, die beispielsweise über die Hubzahl der als Kolbentaumelpumpe ausgebildeten Pumpe 34 oder über einen Durchflussmesser (nicht dargestellt) bestimmbar ist.

Nachdem eine ausreichende Flüssigkeitsmenge in das Behältnis 10 eingeleitet wurde, wird ein Ventil 36 in einer Zuleitung 38 geschlossen. Anschließend wird ein Ventil 40, das in einer die zweite Öffnung 18 mit einem Eingang 42 verbindenden Leitung 44 angeordnet ist, geöffnet, so dass ein Zirkulationskreislauf, bestehend aus der Pumpe 34, einer einen Ausgang 46 der Pumpe mit der ersten Öffnung 16 verbindenden Leitung 48, dem Behältnis 10 und der Leitung 44 gebildet wird. Nachdem das Ventil 36 geschlossen und das Ventil 40 geöffnet ist, wird die Pumpe 34 gestartet, um eine Zirkulation der in dem Behältnis enthaltenen Lösung 52 zu erreichen, damit die vollständige Auflösung der enthaltenen Feststoffe beschleunigt wird. Die Vermischung und Konzentration der Lösung kann über ein im Verlauf der Leitung 48 angeordnetes Leitfähigkeitsmessgerät 52 oder anhand der Zirkulationszeit, insbesondere der Anzahl der Hübe der Kolbentaumelpumpe 34 bestimmt werden.

Nachdem die festen Bestandteile 12 des sauren Konzentrats in der Flüssigkeit vollständig gelöst und homogen verteilt sind, ist in dem Behältnis 10 ein verwendungsfähiges, saures, dialysefähiges Konzentrat entstanden. Nach Öffnen eines Ventils 54 kann das Dialysekonzentrat 52 über die erste Öffnung 16, die Leitung 48 und gegebenenfalls eine weitere Pumpe 56 in einem Verhältnis von beispielsweise 1: 34 bzw. 1: 44 dem Dialysegerät (nicht dargestellt) zugeführt werden.

## Patentansprüche

1. Behältnis (10) mit Bestandteilen (12) eines Konzentrats, insbesondere mit Bestandteilen eines sauren Konzentrats für eine Dialysierflüssigkeit, wobei das Behältnis (10) eine erste und eine zweite Einlass-/Auslassöffnung (16, 18) aufweist,
**dadurch gekennzeichnet,**
**dass** das Behältnis (10) selbst als Mischbehältnis mit einem Volumen derart ausgebildet ist, dass eine definierte Flüssigkeitsmenge zur Lösung der Bestandteile (12) aufnehmbar ist.

2. Behältnis nach Anspruch 1,
**dadurch gekennzeichnet,**
**dass** das Behältnis (10) einen im Wesentlichen trichterförmigen Bereich (14) zur Aufnahme der Bestandteile (12) des Konzentrats aufweist, an dessen spitz zulaufender Grundseite (20) die erste Einlass-/Auslassöffnung (16) für die Flüssigkeit und/oder die Konzentratlösung angeordnet ist.

3. Behältnis nach Anspruch 1 oder 2.
**dadurch gekennzeichnet,**
**dass** die zweite Einlass-/Auslassöffnung (18, 26) bezogen auf die Grundseite (20) der trichterförmigen Aufnahme in einer Höhe angeordnet ist, die unterhalb eines Flüssigkeitspegels des mit Flüssigkeit gefüllten Behältnisses angeordnet ist.

4. Behältnis nach zumindest einem der vorhergehenden Ansprüche,
**dadurch gekennzeichnet,**
**dass** das vorzugsweise als Einwegartikel ausgebildete Behältnis (10) wie Kunststoffbeutel ein Volumen bzw. Fassungsvermögen von 3 1 bis 8 1, insbesondere 5 1 aufweist.

5. Verfahren zum Herstellen eines Konzentrats (50), insbesondere eines sauren Konzentrats für eine Dialysierflüssigkeit, durch Vermischen von in einem Behältnis (10) transportierbaren Bestandteilen (12) mit einer Flüssigkeit,
**dadurch gekennzeichnet,**
**dass** als Behältnis (10) ein solches verwendet wird, in dem die Bestandteile (12) in der Flüssigkeit aufgelöst werden und in dem das fertige Konzentrat bereitgestellt wird.

6. Verfahren nach Anspruch 5,
**dadurch gekennzeichnet,**
**dass** eine definierte Flüssigkeitsmenge in das Behältnis (10) eingeleitet wird und dass die sich in dem Behältnis (12) bildende Lösung (50) sodann beispielsweise mittels einer Pumpe (34) zirkuliert wird, wobei eine Durchmischung bei einer Temperatur von 20 °C bis 39 °C, vorzugsweise bei 37 °C durchgeführt wird.

7. Verfahren nach Anspruch 5 bis 6,
**dadurch gekennzeichnet,**
**dass** die in das Behältnis (10) eingeleitete Flüssigkeitsmenge über eine Steuerung der Pumpe (34) in Abhängigkeit einer Salzmenge in dem Behältnis (10), vorzugsweise über eine Hubzahl der als Kolbentaumelpumpe ausgebildeten Pumpe (34) oder mittels eines Durchflussmessgerätes (52) bestimmt wird und dass das gelöste Konzentrat (50) unmittelbar aus dem Behältnis (10) gegebenenfalls über eine Pumpe (56) einem Dialysegerät zugeführt wird.

8. Vorrichtung zum Herstellen eines Konzentrats (50), insbesondere eines sauren Konzentrats einer Dialysierflüssigkeit, umfassend ein in einem Kreislauf angeordnetes Behältnis (10), in dem Bestandteile (12) des Konzentrats (50) in einer Flüssigkeit zur Bildung des Konzentrats (50) lösbar sind sowie eine in dem Kreislauf angeordnete Pumpe zur Zirkulation der Flüssigkeit,
**dadurch gekennzeichnet,**
**dass** das Behältnis (10) als Transportbehältnis für die Bestandteile (12) ausgebildet ist und ein Volumen derart aufweist, dass eine definierte Flüssigkeitsmenge zur Lösung der Bestandteile aufnehmbar ist.

9. Vorrichtung nach Anspruch 8,
**dadurch gekennzeichnet,**
**dass** ein Ausgang der Pumpe (34) mit der ersten Einlass-/Auslassöffnung (16) des Behältnisses (10) verbunden ist, dass die zweite Einlass-/Auslassöffnung (18) des Behältnisses vorzugsweise über ein Ventil mit einem Eingang (42) der Pumpe (34) verbunden ist und dass des Weiteren der Eingang (42) der Pumpe (34) vorzugsweise über ein Ventil mit einer Flüssigkeitsquelle (32) und die erste Einlass-/Auslassöffnung (16) vorzugsweise über eine weitere Pumpe (46) mit einem Dialysegerät verbunden ist.

10. Vorrichtung nach Anspruch 8 und 9,
**dadurch gekennzeichnet,**
**dass** die Vorrichtung (30) in ein Dialysegerät integriert oder als Zusatzgerät mit einem Dialysegerät verbunden ist.

11. Vorrichtung nach Anspruch 12 bis 15,
**dadurch gekennzeichnet,**
**dass** in dem Zirkulationskreislauf ein Durchflussmesser (52) und/oder ein Leitfähigkeitsmessgerät angeordnet ist.
